**Europäisches Patentamt**

**European Patent Office**

**Office européen des brevets**

(11) Veröffentlichungsnummer: **0 607 811 A1**

# (12) EUROPÄISCHE PATENTANMELDUNG

(21) Anmeldenummer: **94100140.6**

(22) Anmeldetag: **07.01.94**

(51) Int. Cl.5: **C07C 33/04**, C07C 29/42

(30) Priorität: **22.01.93 DE 4301613**

(43) Veröffentlichungstag der Anmeldung:
**27.07.94 Patentblatt 94/30**

(84) Benannte Vertragsstaaten:
**BE DE FR GB NL**

(71) Anmelder: **BASF Aktiengesellschaft
Carl-Bosch-Strasse 38
D-67063 Ludwigshafen(DE)**

(72) Erfinder: **Rittinger, Stefan, Dr.
Danziger Platz 5
D-67059 Ludwigshafen(DE)**
Erfinder: **Rieber, Norbert, Dr.
Liebfrauenstrasse 1c
D-68259 Mannheim(DE)**

(54) **Verfahren zur Herstellung von Hexa-2,4-diin-1,6-diol.**

(57) Verfahren zur Herstellung von Hexa-2,4-diin-1,6-diol der Formel I

$$HO\text{-}CH_2\text{-}C{\equiv}C\text{-}C{\equiv}C\text{-}CH_2\text{-}OH \qquad (I),$$

durch Umsetzung von Diacetylen der Formel II

$$H\text{-}C{\equiv}C\text{-}C{\equiv}C\text{-}H \qquad (II),$$

mit Formaldehyd der Formel III

$$H_2C = O \qquad (III),$$

in der Gegenwart von Silberkatalysatoren, indem man die Umsetzung in Gegenwart eines polaren Lösungsmittels bei Temperaturen von 0 bis 150 °C und Drücken von 0,01 bis 10 bar durchführt.

EP 0 607 811 A1

Die vorliegende Erfindung betrifft ein Verfahren zur Herstellung von Hexa-2,4-diin-1,6-diol durch Umsetzung von Diacetylen mit Formaldehyd in Gegenwart eines polaren Lösungsmittels und katalytischer Mengen eines Silberkatalysators.

Aus der DE-A-877 453 und DE-A-869 053 ist ein Verfahren zur Herstellung von Hexa-2,4-diin-1,6-diol durch Umsetzung von wäßrigen Formaldehydlösungen mit Diacetylen in Gegenwart von Ag-Katalysatoren bekannt. Bei der Verwendung einer Apparatur nach Beispiel 1 der DE-A-877 453 in einer sicher handhabbaren Verdünnung für Diacetylen (DE-A-41 37 011) findet man nur niedrige Umsätze an Diacetylen bei sehr unbefriedigender Reaktionsgeschwindigkeit (Vergleichsbeispiel A). Die in der DE-A-877 453 beschriebene Vorgehensweise zur Verbesserung des Diacetylenumsatzes (Kondensation und Rückführung nicht umgesetzten und ausgegasten Diacetylens) ist aus Sicherheitsgründen im technischen Maßstab nicht praktikabel.

Der vorliegenden Erfindung lag daher die Aufgabe zugrunde, den zuvor genannten Nachteilen abzuhelfen.

Demgemäß wurde ein neues und verbessertes Verfahren zur Herstellung von Hexa-2,4-diin-1,6-diol der Formel I

$$HO-CH_2-C\equiv C-C\equiv C-CH_2-OH \qquad (I),$$

durch Umsetzung von Diacetylen der Formel II

$$H-C\equiv C-C\equiv C-H \qquad (II),$$

mit Formaldehyd der Formel III

$$H_2C=O \qquad (III),$$

in der Gegenwart von Silberkatalysatoren gefunden, welches dadurch gekennnzeichnet ist, daß man die Umsetzung in Gegenwart eines polaren Lösungsmittels bei Temperaturen von 0 bis 150°C und Drücken von 0,01 bis 10 bar durchführt.

Das erfindungsgemäße Verfahren kann wie folgt durchgeführt werden:
Das Formaldehyd III gegebenenfalls in Wasser oder wäßriger Lösung (Formalinlösung) kann mit einem polaren Lösungsmittel in Gegenwart von 0,001 bis 5 Gew.-%, bevorzugt 0,005 bis 2 Gew.-%, besonders bevorzugt 0,01 bis 1 Gew.-% eines Silberkatalysators vorgelegt und das Diacetylen bei Temperaturen von 0 bis 150°C, bevorzugt 20 bis 130°C, besonders bevorzugt 70 bis 110°C und Drücken von 0,01 bis 1,4 bar, bevorzugt 0,1 bis 1,2 bar, besonders bevorzugt bei Atmosphärendruck (Normaldruck) darin eingegast werden.

Als Silberkatalysatoren eignen sich in der Regel elementares, metallisches Silber oder Ag$^+$-Salze, insbesondere Silber oder Silberoxid auf inerten Trägern wie Aluminiumoxid oder Siliciumdioxid.

Das vorliegende Verfahren eignet sich im besonderen zur Verwertung von diacetylenhaltigen Teilströmen, wie sie technisch üblicher Weise bei der Zerlegung des Spaltgases aus der Spaltung von Kohlenwasserstoffen unter den Bedingungen der Acetylensynthese anfallen (Ullmann's Encycl. of Indust. Chem., V. Ed., A1, 1985). Die dabei teils gasförmig, teils flüssig anfallenden Gemische höherer Acetylene lassen sich im erfindungsgemäßen Verfahren ebenfalls verwenden.

Das Gasgemisch aus der Spaltgasaufbereitung hat gewöhnlich gegenüber der Umgebung leicht erhöhte Betriebstemperatur. Die Vorabscheidung von leicht kondensierbaren Gasbestandteilen in Abscheidebehältern in der Reaktorzuleitung bei Umgebungstemperatur verbessert die Reinheit des Rohproduktes.

Die Umsetzung kann sowohl diskontinuierlich als auch bevorzugt kontinuierlich in der Gasphsase oder bevorzugt in der Flüssigphase durchgeführt werden. Bei Nutzung eines gasförmigen Teilstroms sind Verfahren, die bekanntermaßen eine gute Gasverteilung in Flüssigkeiten erzielen, z.B. die Verwendung von Apparateteilen wie Begasungsring, Lochboden, verdichtende Begasungseinrichtungen, Sprühreaktor oder Absorberturm vorteilhaft anzuwenden. Als polare Lösungsmittel eignen sich Lactame, z.B. Pyrrolidone wie N-Methylpyrrolidon, Lactone, z.B. 5- bis 8-Ringlactone wie Butyrolacton, Ester, z.B. $C_1$- bis $C_{20}$-Carbonsäurealkylester wie Ameisensäuremethylester, Essigsäuremethylester, Propionsäuremethylester, Ameisensäureethylester, Essigsäureethylester und Propionsäureethylester, Säureamide, z.B. Dialkylformamide wie Dimethylformamid, Alkohole, z.B. $C_1$- bis $C_{20}$-Alkanole wie Methanol und Ethanol, alkylierte Harnstoffe oder Glykolether wie Ethylenglykoldiethylether.

Hexa-2,4-diin-1,6-diol I ist ein mögliches Vorprodukt von Hexan-1,6-diol, welches ein wichtiges Zwischenprodukt für die Weiterverarbeitung zu Polyestern, Polyurethanen, Klebstoffen, Pharmazeutika und Textilhilfsmittel darstellt.

Beispiele

Das Diacetylen im Gasstrom vor bzw. nach der Reaktion wurde gaschromatographisch auf einer gepackten Säule (20 % Reoplex 400 auf Chromosorb PAW) mit Trägergas $N_2$ (35 ml/min.) und FID-Detektion bestimmt. Die Konzentrationen sind in Vol-% angegeben. Die Diacetylenbestimmung in flüssiger Phase sowie die Ermittlung der Produktgehalte erfolgte gaschromatographisch auf einer Kapillarsäule HP1 mit einem WLD-Detektor.

Beispiel 1

Eine Blasensäule mit einem Durchmesser von 25 mm, einer Höhe von 1000 mm und einer Glasfritte (Porengröße 40 - 90 um) zur Verteilung des eingeleiteten Gases am Fuß der Säule wurde mit einer Schüttung von 150 g Katalysatorringen (13.7 % elementares Ag auf $\alpha$-$Al_2O_3$-Ringen) und mit einer Mischung aus 100 g Formalin (36 %ig) und 100 g N-Methylpyrrolidon (NMP) befüllt. Bei 95 °C Reaktionstemperatur wurden 20 l/h HA-Gas in die Lösung eingeleitet. Aus dem Abgas wurden Flüssigbestandteile kondensiert und in die Reaktion zurückgetropft. Die Hexadiindiolkonzentration stieg linear gegen die Zeit an bis zu einer Konzentration von 9.3 Gew.-% nach 10 h entsprechend einer RZA von ca. 15 g Produkt pro kg Katalysator pro Stunde. Wegen der geringen Restformaldehydkonzentration (< 5 %) ließ die Reaktionsgeschwindigkeit dann nach. Die durchschnittliche Diacetylenabreicherung lag während der ersten 10 Versuchsstunden bei ca. 30 %.

Beispiel 2

Eine Blasensäule mit einem Durchmesser von 25 mm, einer Höhe von 1000 mm und einer Glasfritte (Porengröße 40 - 90 $\mu$m) zur Verteilung des eingeleiteten Gases am Fuß der Säule wurde mit einer Schüttung von 150 g Katalysatorringen (13.7 % elementares Ag auf $\alpha$-$Al_2O_3$-Ringen) befüllt und 200 g des angegebenen Lösungsmittelgemisches (Formalin wurde jeweils als 36 %ige Lösung eingesetzt) und 150 g Katalysator (angegeben: Aktivmassenanteil und Trägermaterial) vorgelegt und bei 95oC 20 l/h HA-Gas eingeleitet. In der Tabelle ist die Abhängigkeit der Raumzeitausbeute (RZA) an Hexadiindiol vom Lösungsmittel für verschiedene Katalysatoren aufgezeigt.

Tabelle
Abhängigkeit der Hexadiindiolbildung vom Lösungsmittel

| Lösungsmittelgemisch | Volumenverhältnis | Katalysator | Raum/Zeit-Ausbeute [g Produkt/kg (Kat) x h] |
|---|---|---|---|
| Wasser/Hexan-1,6-diol | 1 : 1 | 15 Gew.% Ag/$Al_2O_3$ | 4 |
| Wasser/Butyrolacton | 1 : 1 | 15 Gew.% Ag/$Al_2O_3$ | 5 |
| Wasser/Ethylenglykolmonomethylether | 1 : 1 | 15 Gew.% Ag/$Al_2O_3$ | 2 |
| Wasser/N-Methylpyrrolidon | 1 : 3 | 15 Gew.% Ag/$Al_2O_3$ | 5 |
| Wasser/Dimethylformamid | 1 : 1 | 15 Gew.% Ag/$Al_2O_3$ | 6 |
| Wasser/N-Methylpyrrolidon | 1 : 1 | 10 Gew.% Ag/$SiO_2$ | 15 |
| Wasser/N-Methylpyrrolidon | 1 : 1 | 5 Gew.% $AgO_2$/$Al_2O_3$ | 5 |

Vergleichsbeispiel A

Eine Blasensäule mit einem Durchmesser von 25 mm, einer Höhe von 1000 mm und einer Glas-

fritte (Porengröße 40 - 90 $\mu$m) zur Verteilung des eingeleiteten Gases am Fuß der Säule wurde mit einer Schüttung von 150 g Katalysatorringen (13.7 % elementares Ag auf $\alpha$-Al$_2$O$_3$-Ringen) befüllt. Nach Zugabe von 150 g Formalin (36 % Formaldehydgehalt) wurden bei 95°C Reaktionstemperatur 20 l/h HA-Gas in die Lösung eingeleitet. Aus dem Abgas wurden mitgerissene Flüssigbestandteile kondensiert und in die Reaktion zurück getropft. Während 30 h Reaktionszeit betrug die maximale Diacetylenabreicherung 20 %. Im Durchschnitt lag die Abreicherung unter 10 %. Während dieser Zeit stieg die Konzentration an gebildetem Hexadiindiol (I) auf 1.3 Gew.-% entsprechend einer RZA von 0.45 g pro kg Katalysator pro Stunde.

**Patentansprüche**

1.  Verfahren zur Herstellung von Hexa-2,4-diin-1,6-diol der Formel I

    HO-CH$_2$-C$\equiv$C-C$\equiv$C-CH$_2$-OH      (I),

    durch Umsetzung von Diacetylen der Formel II

    H-C$\equiv$C-C$\equiv$C-H     (II),

    mit Formaldehyd der Formel III

    H$_2$C$=$O     (III),

    in der Gegenwart von Silberkatalysatoren, dadurch gekennnzeichnet, daß man die Umsetzung in Gegenwart eines polaren Lösungsmittels bei Temperaturen von 0 bis 150°C und Drücken von 0,01 bis 10 bar durchführt.

2.  Verfahren zur Herstellung von Hexa-2,4-diin-1,6-diol I nach Anspruch 1, dadurch gekennzeichnet, daß man die Umsetzung bei bei Temperaturen von 20 bis 130°C durchführt.

3.  Verfahren zur Herstellung von Hexa-2,4-diin-1,6-diol I nach Anspruch 1, dadurch gekennzeichnet, daß man die Umsetzung bei Temperaturen von 70 bis 110°C durchführt.

4.  Verfahren zur Herstellung von Hexa-2,4-diin-1,6-diol I nach Anspruch 1, dadurch gekennzeichnet, daß man die Umsetzung bei Drücken von 0,1 bis 1,5 bar durchführt.

5.  Verfahren zur Herstellung von Hexa-2,4-diin-1,6-diol I nach Anspruch 1, dadurch gekennzeichnet, daß man die Umsetzung bei Atmosphärendruck durchführt.

6.  Verfahren zur Herstellung von Hexa-2,4-diin-1,6-diol I nach Anspruch 1, dadurch gekennzeichnet, daß man als polare Lösungsmittel Lactame, Lactone, Ester, Säureamide, Alkohole, alkylierte Harnstoffe und/oder Glykolether einsetzt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| D,X, Y | DE-C-877 453 (CHEMISCHE WERKE HÜLS) 26. Mai 1953 <br> * das ganze Dokument * <br> --- | 1-6 | C07C33/04 <br> C07C29/42 |
| X,Y | DE-A-19 06 051 (BASF) <br> * Seite 1 - Seite 6 * <br> --- | 1-6 | |
| X,Y | DE-C-725 326 (I.G.FARBENINDUSTRIE) <br> * das ganze Dokument * <br> --- | 1-6 | |
| Y | CA-A-850 153 (BASF) <br> * Seite 3, Zeile 11 - Zeile 17; Ansprüche; Beispiele * <br> --- | 1-6 | |
| Y | DE-B-11 74 765 (ALLIED CHEMICAL CORPORATION) <br> * Spalte 1 - Spalte 2 * <br> ----- | 1-6 | |

|  | | | RECHERCHIERTE SACHGEBIETE (Int.Cl.5) |
|---|---|---|---|
| | | | C07C |

Der vorliegende Recherchenbericht wurde für alle Patentansprüche erstellt

| Recherchenort | Abschlußdatum der Recherche | Prüfer |
|---|---|---|
| DEN HAAG | 10. März 1994 | Heywood, C |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
....................................................................
& : Mitglied der gleichen Patentfamilie, übereinstimmendes
    Dokument

EPO FORM 1503 03.82 (P04C03)